# EUROPÄISCHE PATENTSCHRIFT

(11) **EP 3 098 305 B1**
(45) Veröffentlichungstag und Bekanntmachung des Hinweises auf die Patenterteilung: **03.10.2018**
(21) Anmeldenummer: 16178934.2
(22) Anmeldetag: 13.08.2008
(51) Int. Cl.: C12N 5/00, C07K 14/435, A61K 47/42

(54) **KOLLAGENHALTIGER ZELLTRÄGER**
CELL CARRIER CONTAINING COLLAGEN
SUPPORT CELLULAIRE CONTENANT DU COLLAGENE

(30) Priorität: 20.08.2007 DE 102007040370
(43) Veröffentlichungstag der Anmeldung: 30.11.2016
(62) Teilanmeldung aus: 08801567.2
(73) Patentinhaber: Eberhard-Karls-Universität Tübingen, 72076 Tübingen (DE)
(72) Erfinder: Just, Lothar, 72076 Tübingen (DE); Schmidt, Timo, 70197 Stuttgart (DE); Maser, Franz, 68159 Mannheim (DE)
(74) Vertreter: Witte, Weller & Partner Patentanwälte mbB

(56) Entgegenhaltungen:
- WO-A-01/92322
- US-A1- 2005 208 478
- MIYATA T ET AL: "Collagen engineering for biomaterial use", CLINICAL MATERIALS, ELSEVIER, GB, Bd. 9, Nr. 3-4, 1. Januar 1992 (1992-01-01), Seiten 139-148, XP023261557, ISSN: 0267-6605 [gefunden am 1992-01-01]
- LEE C H ET AL: "Biomedical applications of collagen", INTERNATIONAL JOURNAL OF PHARMACEUTICS, ELSEVIER BV, NL, Bd. 221, Nr. 1-2, 19. Juni 2001 (2001-06-19), Seiten 1-22, XP002346259, ISSN: 0378-5173

## Beschreibung

Die vorliegende Erfindung betrifft die Verwendung einer kollagenhaltigen Zusammensetzung zur Kultivierung von biologischen Zellen.

Träger zur Kultivierung von biologischen Zellen sind im Stand der Technik allgemein bekannt. Solche Träger werden häufig als Matrix oder Scaffold bezeichnet. Diese Träger stellen den Nährboden oder allgemein den Untergrund dar, auf dem die Zellen in Zellkultur aufsitzen.

Kollagenhaltige Zusammensetzungen stellen den bekanntesten Typ von Zellträgern dar. Kollagen ist ein zu den Skleroproteinen zählendes, in der Regel wasserunlösliches, faserig aufgebautes, tierisches Protein der extrazellulären Matrix, das besonders am Aufbau von Bindegeweben, z.B. der Haut, Blutgefäße, Bänder, Sehnen und Knorpel, sowie am Aufbau von Knochen und Zähnen beteiligt ist. Aufgrund dieser Eigenschaften werden kollagenbasierende Biomaterialien tierischer Herkunft bereits seit Jahren in der Medizin eingesetzt. Insbesondere in klinisch anwendbaren Produkten zur Blutstillung, als Dura-Ersatz oder auch in verschiedenen Bereichen der plastischen Chirurgie konnten sich Kollagene als Trägermaterial etablieren. Kollagene, von denen bis heute 28 verschiedene Typen identifiziert wurden und mindestens 10 weitere Proteine mit kollagenähnlichen Domänen zu verzeichnen sind, unterscheiden sich zwischen den einzelnen Spezies nur geringfügig. Da kein ausgeprägtes Identifikationsmuster vorhanden ist und der durch Enzyme bestimmte Abbau keine toxischen Abbauprodukte hervorbringt, gelten Kollagene als biokompatibel.

Die bisher am Markt angebotenen Kollagenmatrices zeigen eine sehr große Varianz in ihren Eigenschaften. So wurde festgestellt, dass einige der zur Kultivierung von biologischen Zellen eingesetzten Kollagenmatrices *in vitro* Entzündungsreaktionen auslösen, die katabole Stoffwechselprozesse zur Folge haben. Ein weiterer Nachteil der momentan zur Verfügung stehenden Kollagenmatrices liegt darin, dass diese sehr dick sind, in der Regel größer als 200 µm und deshalb nicht mikroskopierbar sind. Hinzu kommt, dass die bisher zur Kultivierung von biologischen Zellen angebotenen Kollagenmatrices sehr teuer sind.

Als Träger zur Kultivierung von Zellen kommen ferner so genannte Hydrogele in Frage. Ein Hydrogel ist ein Wasser enthaltendes, aber wasserunlösliches Polymer, dessen Moleküle chemisch, z.B. durch kovalente oder ionische Bindungen, oder physikalisch, z.B. durch Verschlaufen der Polymerketten, zu einem dreidimensionalen Netzwerk verknüpft sind. Aufgrund von eingebauten hydrophilen Polymerkomponenten quellen sie in Wasser unter beträchtlicher Volumenzunahme, ohne aber ihren stofflichen Zusammenhalt zu verlieren. Nachteilig bei Hydrogelen ist jedoch, dass diese aufgrund der enormen Wasserspeicherung mechanisch instabil sind. Häufig kommt es während der Besiedlung mit Zellen ferner zu einer Kondensierung der Hydrogele. Hydrogele sind deshalb in ihrer Anwendung sehr begrenzt.

Eine weitere, zur Besiedlung mit biologischen Zellen geeignete Matrix stellt die so genannte Hyaluronsäure dar. Diese Matrix besteht aus Makromolekülen der Knorpelgrundsubstanz und weist deshalb in unmodifizierter Form eine hohe Biokompatibilität auf. Um eine geeignete Struktur mit ausreichender mechanischer Belastbarkeit zu generieren, müssen die Molekülketten vernetzt werden. Diese Vernetzung geschieht durch Veresterung mit Alkohol, was zu einer Beeinträchtigung der Biokompatibilität führen kann.

Ein weiteres Scaffold stellt das so genannte Alginat dar. Hierbei handelt es sich um ein aus Braunalgen gewonnenes Copolymer, das sich aus L-Guluronsäure und D-Mannuronsäure zusammensetzt. Durch die Zugabe von EDTA bzw. Na-Citrat kann das Produkt geliert oder verflüssigt werden. Dies verleiht dem Alginat ähnliche Eigenschaften für die Zellkultivierung wie sie bereits für kollagenbasierende Gele beschrieben wurden, jedoch mit verbesserter Resuspensionsmöglichkeit für zell- und molekularbiologische Analysen. Trotz der Vorteile gegenüber anderen Trägermatrices und guten Eigenschaften des Materials *in vitro* konnten *in vivo* keine Erfolge erzielt werden. Die Substanz ist *in vivo* schlecht resorbierbar und verursacht erhebliche Immun- und Fremdkörperreaktionen. Alginat ist deshalb bislang nicht als Trägermaterial für Implantate am Menschen verwendbar.

Ein weiteres Trägermaterial für biologische Zellen ist Agarose. Sie verhält sich ähnlich wie Alginat. Agarose besteht aus zwei Zuckerketten und wird aus den Zellwänden von Rotalgen gewonnen. Wie Alginat verursacht es *in vivo* Immun- und Fremdkörperreaktionen, so dass Agarose bislang nicht am Menschen eingesetzt werden konnte.

Weitere Scaffolds basieren auf Fibrin. Hierbei handelt es sich um ein globuläres Plasmaprotein, das durch seine Fähigkeit zur vernetzenden Polymerisation u.a. die Blutgerinnung bewirkt. Auch Fibrin hat sich bislang jedoch nicht für eine Verwendung *in vivo* bewährt. Die Verwendung von Fibrin als Zellträger ist weitgehend unbeforscht und muss noch ausgiebig evaluiert werden. Fibrin ist darüber hinaus sehr teuer.

Weitere, zur Kultivierung von biologischen Zellen verwendete Matrices basieren auf Chitin bzw. Chitosan. Chitin bildet den Ausgangsstoff zur Chitosanherstellung. Dazu werden die Acetylgruppen des Chitins chemisch oder enzymatisch abgespalten. Sowohl bei Chitin als auch bei Chitosan handelt es sich um Biopolymere, zwischen denen es keinen genau definierten Übergang gibt. In der Regel wird aber von Chitosan gesprochen, wenn der Deacetylierungsgrad größer 40 - 50 % ist und die Verbindung in organischen Säuren löslich ist. Chitin bzw. Chitosan sind jedoch in ihren Anwendungsmöglichkeiten stark eingeschränkt und haben sich in der Praxis zur Kultivierung von Zellen bislang ebenfalls nicht bewährt. Chitin ist kein körpereigener Stoff und ist daher stets ein Fremdkörper im Organismus. Die Verwendung von Chitin als Zellträger muss noch grundlegend beforscht werden.

Derzeit befinden sich auch eine Vielzahl von künstlichen Scaffolds in der Erprobung. Zu diesen zählen die Polymere Polyactide (PLA) und Polyglycolide (PGA) sowie Poly-L-Milchsäure (engl.: "poly-L-lactic acid", PLLA bzw. "bioglass"). Besonderes Kennzeichen der Polymere PLA und PGA ist ihre geringe Löslichkeit in wässrigen Medien, die sich erst durch den Polymerkettenabbau, d.h. die Hydrolyse, zu niedermolekularen Oligomeren oder Monomeren verbessert und damit zur Erosion dieser Materialien führt. Allerdings hat sich gezeigt, dass diese Polymere nicht zur Kultivierung von beliebigem biologischem Material geeignet sind. Resorbierbare Polymere zerfallen durch spontane Hydrolyse in organische Säuren. Osteoblasten differenzieren in saurem Milieu, so dass eine hohe Menge dieser Polymere eher zu Knochenabbau als zu Knochenaufbau führen kann.

Dokument US2005/0208478 offenbart ein zweischichtiges kollagenhaltiges Kompositprodukt, das nach Erkenntnissen der Erfinder extrem feuchtigkeitsarm und fragil ist. Es ist deshalb sehr schlecht handhabbar und für Anwendungen im modernen "Tissue Engineering" ungeeignet.

Vor diesem Hintergrund liegt der Erfindung die Aufgabe zugrunde, eine neue Zusammensetzung zur Kultivierung von biologischen Zellen bereitzustellen, mit der die Nachteile vermieden werden, die sich bei dem im Stand der Technik bekannten Zellträgern zeigen. Insbesondere soll eine solche Zusammensetzung bereitgestellt werden, die im Großmaßstab kostengünstig und in gleichbleibend hoher Qualität herstellbar ist.

Diese Aufgabe wird durch die Verwendung einer Zusammensetzung zur Kultivierung von biologischen Zellen gelöst, die folgende Parameter aufweist:

| | |
|---|---|
| Kollagen [Gew.-%]: | ca. 30 bis 80, |
| Amidstickstoff [Gew.-%]: | ca. 0,06 bis 0,6, |
| Polyol [Gew.-%]: | ca. 0 bis 50, |
| Fett [Gew.-%]: | ca. 0 bis 20, |
| Asche [Gew.-%]: | ca. 0 bis 10, |
| Wasser [Gew.-%]: | ca. 5 bis 40, |
| pH-Wert: | ca. 3 bis 10, |
| Flächengewicht [g/m²]: | ca. 10 bis 100, |
| Reißfestigkeit [N/mm²]: | ca. 0,5 bis 100, bzw. 20 bis 100. |

Eine solche Zusammensetzung ist in Folienform bei der Firma Naturin GmbH & Co. KG, Badeniastraße 13, Weinheim, bereits kommerziell erhältlich. Diese Folien haben beispielsweise die von der Firma Naturin zugeordneten Referenznummern 400011899, 400023747, 400024203, 400026193, 400019485, 400000084 und 400000109.

Diese Erkenntnis war überraschend. Diese Zusammensetzung wurden bisher ausschließlich in der Lebensmittelbranche verwendet, bspw. als Trennfolie zwischen Netz und Fleisch im Rahmen der Schinkenherstellung. Es war insbesondere nicht zu erwarten, dass derartige Zusammensetzungen zur Kultivierung von biologischem Material besonders geeignet sind.

Die erfindungsgemäße Zusammensetzung lässt sich im Großmaßstab reproduzierbar herstellen und ist von gleichbleibend hoher Qualität. Die Zusammensetzung zeichnet sich insbesondere durch ihre gute Biokompatibilität, ihre sehr dünne Folienstärke, ihre verhältnismäßig hohe Transparenz und ihre hohe mechanische Stabilität aus, so dass sich ein breites Anwendungsspektrum ergibt.

Dabei ist es bevorzugt, wenn als Polyol Glycerin und/oder Sorbit verwendet wird, welches in einer Konzentration von 0 bis 50 Gew.-% (Glycerin), bzw. 0 bis 40 Gew.-% (Sorbit) vorgesehen ist.

Diese Maßnahme hat den Vorteil, dass Polyole zum Einsatz kommen, die sich in den genannten Konzentrationen als Feuchthalte- bzw. Wasserbindemittel zum Schutz vor Austrocknung besonders bewährt haben.

Nach einer Variante weist die Zusammensetzung folgende Parameter auf:

| | |
|---|---|
| Kollagen [Gew.-%]: | ca. 50 bis 70, |
| Amidstickstoff [Gew.-%]: | ca. 0,14 bis 0,4 |
| Glycerin [Gew.-%]: | ca. 12 bis 35, |
| Fett [Gew.-%]: | ca. 3 bis 7, |
| Sorbit [Gew.-%]: | ca. 0 bis 20, |
| Asche [Gew.-%]: | ca. 0,5 bis 3, |
| Wasser [Gew.-%]: | ca. 12 bis 18, |
| pH-Wert: | ca. 5,5 bis 8, |
| Flächengewicht [g/m²]: | ca. 20 bis 40, |
| Reißfestigkeit [N/mm²]: | ca. 5 bis 25 bzw. 40 bis 80. |

Mit dieser Maßnahme werden die Konzentrationen der jeweiligen Bestandteile nochmals optimiert, so dass die Zusammensetzung in ihrer Eignung zur Kultivierung von biologischen Zellen nochmals verbessert wird.

Dabei ist es bevorzugt, wenn das Fett im Wesentlichen pflanzliches Öl ist.

Die Verwendung von pflanzlichem Öl zum Erhalt der erfindungsgemäßen Zusammensetzung hat sich besonders vorteilhaft herausgestellt. So verbessert pflanzliches Öl durch Erhöhung der Dehnbarkeit die Einsatzmöglichkeiten der Zusammensetzung deutlich. Auch können durch Verwendung von pflanzlichem Öl Ranzigkeitserscheinungen vermieden und somit die Herstellung und Lagerung der Zusammensetzung verbessert werden. Es versteht sich, dass ein minimaler Restanteil tierischen Fetts enthalten sein kann, ohne dass die Vorteile des pflanzlichen Öls aufgehoben werden.

Nach einer besonders bevorzugten Ausgestaltung liegt der pH-Wert der erfindungsgemäßen Zusammensetzung bei ca. 5,0 bis 8,0, vorzugsweise 6,8 bis 8,0, weiter vorzugsweise bei 7,0 bis 7,8, höchst bevorzugt bei ca. 7,2 bis 7,5.

Wie die Erfinder herausgefunden haben, gelingt eine Kultivierung von biologischen Zellen bei den genannten pH-Werten besonders gut. Somit weist die Zusammensetzung einen pH-Wert auf, der im physiologischen Bereich liegt und deshalb ein Milieu bereitstellt, das der natürlichen Umgebung der biologischen Zellen weitgehend ähnelt. Die kommerziell erhältlichen Zusammensetzungen, bspw. der Firma Naturin GmbH & Co. KG, haben in der Regel einen pH-Wert, der bei etwa 4,8 liegt. Unter derartig sauren Bedingungen ist bspw. eine Kultivierung von säureempfindlichem biologischem Material nicht möglich. Die Einstellung des gewünschten pH-Wertes kann durch Inkubation der Zusammensetzung in bspw. calcium- oder magnesiumhaltigem Phosphatpuffer erfolgen, wobei andere, dem Fachmann bekannte, übliche Puffer gleichermaßen geeignet sind.

Vor diesem Hintergrund betrifft ein weiterer Gegenstand der vorliegenden Offenbarung auch ein Verfahren zur Verbesserung einer Zusammensetzung, die folgende Parameter aufweist:

| | |
|---|---|
| Kollagen [Gew.-%]: | ca. 30 bis 80, |
| Amidstickstoff [Gew.-%]: | ca. 0,06 bis 0,6, |
| Polyol [Gew.-%] | ca. 0 bis 50, |
| Fett [Gew.-%]: | ca. 0 bis 20, |
| Asche [Gew.-%]: | ca. 0 bis 10, |
| Wasser [Gew.-%]: | ca. 5 bis 40, |
| pH-Wert: | ca. 3 bis 10, |
| Flächengewicht [g/m²]: | ca. 10 bis 100, |
| Reißfestigkeit [N/mm²]: | ca. 0,5 bis 100 bzw. 20 bis 100, |

in ihrer Eignung zur Kultivierung von biologischen Zellen, das folgende Schritte aufweist:
(1) Bereitstellung der Zusammensetzung, und
(2) Einstellung des pH-Wertes auf ca. 5,0 bis 8,0, vorzugsweise ca. 6,8 bis 8,0, weiter vorzugsweise auf ca. 7,0 bis 7,8, höchst bevorzugt auf ca. 7,2 bis 7,5.

Mit diesem "Verbesserungsverfahren" lassen sich kommerziell erhältliche Folien, wie bspw. jene, die von der Firma Naturin GmbH & Co. KG angeboten werden, auf einfache Art und Weise hinsichtlich ihrer Eignung als Zellkulturträger, deutlich verbessern. Schritt (2) erfolgt dabei bevorzugt durch die Inkubation der Zusammensetzung in Pufferlösung mit einem pH-Wert von ca. 7,2 bis 7,5.

Das Verbesserungsverfahren weist bevorzugt einen weiteren Schritt (3) auf, in dem die Zusammensetzung mit stark fettlöslichen Substanzen inkubiert wird.

Diese Maßnahme hat den Vorteil, dass bspw. durch Verwendung von 100 %igem Azeton fettlösliche Substanzen extrahiert werden. Die Eigenschaft der Zusammensetzung zur Kultivierung von biologischem Material kann dadurch nochmals verbessert werden.

Weiter ist es bei dem Verbesserungsverfahren bevorzugt, wenn Schritt (3) den Schritt (3.1) umfasst, in dem ein Waschen der Zusammensetzung in Pufferlösung erfolgt.

Mit diesem Schritt (3.1) wird der Rest an stark fettlöslichen Substanzen und ggf. weitere kontaminierende Rückstände entfernt, so dass dann die Membran einsatzbereit ist bzw. weiter ver- oder bearbeitet werden kann.

Das Verbesserungsverfahren weist bevorzugt einen weiteren Schritt (4) auf, in dem die Trocknung der Zusammensetzung erfolgt.

Diese Maßnahme hat den Vorteil, dass dadurch ein Produkt erhalten wird, welches sich einfach handhaben und nahezu unbegrenzt lagern lässt.

Nach einer bevorzugten Ausgestaltung ist die erfindungsgemäße Zusammensetzung als Flachfolie ausgebildet.

Mit dieser Maßnahme wird die Zusammensetzung in einer Form bereitgestellt, die sich sowohl für Zellkultivierungsanwendungen als auch für die Implantation von biologischem Material in einen Organismus besonders eignet. Die als Folie ausgebildete erfindungsgemäße Zusammensetzung lässt sich einfach in eine beliebige Form und Größe schneiden oder stanzen.

Vor diesem Hintergrund ist die Zusammensetzung bevorzugt als Träger oder Matrix bzw. "Scaffold" für Zellkultivierungsanwendungen bzw. als Träger für die Implantation von biologischem Material, vorzugsweise von Stamm- und Vorläuferzellen, oder gewebespezifischen Zellen, in einen Organismus, ausgebildet. Sie ist wegen ihrer Biokompatibilität und der adhärenzfördernden Eigenschaft zur Immobilisierung von Zellen nutzbar. Dies kann im Bereich der regenerativen Medizin zum Beispiel zur Zucht von Bändern, Sehnen, Knochen und Knorpel von hoher Relevanz sein, darüber hinaus aber auch auf weitere Gewebe angewandt werden. Sie ist deshalb auch als Wundauflage geeignet. Weiter kann die Zusammensetzung z.B. im Kosmetikbereich als Hautauflage eingesetzt werden. Darüber hinaus eignet sie sich auf Grund der chemischen Zusammensetzung der Kollagene im Besonderen zur Kopplung von zellbeeinflussenden Wirkstoffen, wie z.B. Wachstumsfaktoren.

Besonders vorteilhaft ist, dass die erfindungsgemäße Flachfolie nach dem Trocken ohne weitere Fixierungshilfen auf glatte Kunststoffoberflächen adhäriert. Die Folie bildet somit bspw. mit Zellkulturplastikoberflächen eine luftblasenfreie Einheit, die auch bei schonender Zellkultivierung erhalten bleibt. Auf zellbeeinflussende Klebe- und Fixierungshilfen kann daher in der Zellkultur verzichtet werden. Für spezifische Applikationen lässt sich diese Verbindung jedoch beschädigungsfrei mit mechanischen Hilfen, wie z.B. Pinzetten lösen und die Folie aus der Zellkulturschale mit den auf ihr wachsenden Zellen entnehmen.

Alternativ ist die erfindungsgemäße Zusammensetzung als Schlauchhülle ausgebildet.

Diese Ausgestaltung ist besonders als Zell- und Wirkstoffreservoir für Implantationsversuche geeignet.

Die erfindungsgemäße Flachfolie bzw. Schlauchhülle weist in getrocknetem Zustand eine Dicke auf, die bei ca. 5 bis 200 µm, vorzugsweise 10 bis 100 µm, weiter bevorzugt bei 15 bis 30 µm, weiter bevorzugt bei ca. 20 µm und höchst bevorzugt bei ca. 15 µm liegt.

Diese Maßnahme hat den Vorteil, dass eine besonders dünne Folie bzw. Hülle bereitgestellt wird, die transparent und dadurch mikroskopierbar ist. Dies ist bei den derzeit im Stand der Technik bekannten, auf Kollagen basierenden Zellträgern nicht der Fall. Die Dicke wird erfindungsgemäß in getrocknetem Zustand bestimmt. Getrocknet heißt hierbei luftgetrocknet, so dass eine absolute Restfeuchte verbleibt, die bei zwischen ca. 10 % und 15 % liegt.

Nach einer bevorzugten Weiterbildung ist die Zusammensetzung strahlungssterilisiert, was vorzugsweise durch ionisierende Strahlung, weiter bevorzugt durch Beta-Strahlung und/oder Gamma-Strahlung erfolgt.

Diese Maßnahme hat den Vorteil, dass kontaminierende Organismen abgetötet und Kontaminationen des zu kultivierenden biologischen Materials weitgehend vermieden werden. Die Strahlungssterilisation bietet den Vorteil, dass dadurch das wärmeempfindliche Kollagen unbeschadet bleibt.

Vor diesem Hintergrund weist das Verbesserungsverfahren den weiteren Schritt (5) auf, in dem die Strahlungssterilisation der Zusammensetzung erfolgt.

Weiter ist es bevorzugt, wenn die erfindungsgemäße Zusammensetzung einen Farbstoff, vorzugsweise eine fluoreszenzabsorbierenden Farbstoff, aufweist.

Für die *In-vitro*-Diagnostik kann die Zusammensetzung unterschiedlich gefärbt werden. Hierbei können bei so genannten Penetrationstests für pharmakologische, physiologische oder zellbiologische Untersuchungen fluoreszenzgefärbte Zellen nachgewiesen werden, die durch die Folie penetriert sind, da eine beispielsweise fluoreszensabsorbierende Färbung der Zusammensetzung die fluoreszierenden Zellen, die nicht penetriert sind, verdeckt. Nur die penetrierten Zellen leuchten dann bei der fluoreszensmikroskopischen Untersuchung.

Ein weiterer Gegenstand der vorliegenden Offenbarung ist ein Verfahren zur Kultivierung von biologischem Material, das folgende Schritte aufweist:
(1) Bereitstellung einer als Träger für biologisches Material geeigneten Zusammensetzung,
(2) Inkontaktbringen von biologischem Material mit der Zusammensetzung, und
(3) Inkubation der Zusammensetzung mit dem biologischen Material unter Kultivierungsbedingungen,
wobei als Zusammensetzung die vorstehend im Zusammenhang mit der erfindungsgemäßen Verwendung beschriebene Zusammensetzung verwendet wird.

Ein weiterer Gegenstand der vorliegenden Offenbarung betrifft ein Verfahren zur Implantation von biologischem Material in einen Organismus, das folgende Schritte aufweist:
(1) Bereitstellung einer als Träger für biologisches Material geeigneten Zusammensetzung,
(2) Inkontaktbringen von biologischem Material mit der Zusammensetzung, und
(3) Einbringen des biologischen Materials in Kontakt mit der Zusammensetzung in einen Organismus,
wobei als Zusammensetzung die vorstehend im Zusammenhang mit der erfindungsgemäßen Verwendung beschriebene Zusammensetzung verwendet wird.

Ein weiterer Gegenstand der vorliegenden Offenbarung betrifft die Verwendung der erfindungsgemäßen Zusammensetzung zur Bestimmung des Invasions- und Metastasierungspotential von Tumorzellen.

Gängige *in vitro*-Testverfahren zum Invasions- und Metastasierungspotential von Tumorzellen basieren auf folgendem Prinzip: Gemessen wird die Penetrationsleistung von Tumorzellen durch eine perforierte nicht abbaubare Kunststofffolie. In *in vitro-*Tests werden mit diesem System zum Beispiel die Wirkung von antikarzinogenen Pharmaka auf die Penetrationsleistung der Zellen gemessen. Die Invasivität einer Tumorzelle hängt aber nicht nur von der Migrationsleistung durch Lücken (Poren) im Bindegewebe ab, sondern auch von der Fähigkeit der Zellen, die Bindegewebskomponenten, zu den im großen Maße die Kollagene zählen, enzymatisch ab- bzw. umzubauen. Mit Hilfe der biologischen Kollagenfolie könnte auf Grund ihrer geringen Dicke, Homogenität und standardisierten Herstellung ein neues Pharma-Testsystem aufgebaut werden, um die proteolytische Leistung von kultivierten Zellen zu bestimmen. Für dieses System werden die Zellen in einem Zweikammersystem kultiviert. Beide Kammern werden durch die Kollagenfolie getrennt. Die zu untersuchenden Zellen werden in der oberen Kammer auf der Folie kultiviert. Nach entsprechender Kulturdauer wird die Anzahl der durch die Kollagenmembran migrierten Zellen auf der Membranunterseite quantifiziert. Mit Hilfe der erfindungsgemäßen Zusammensetzung erschließt sich deshalb ein neues Feld in der *In*-*vitro*-Diagnostik.

Die erfindungsgemäße Zusammensatzung kann auch dazu verwendet werden, um die proteolytische Aktivität von Nicht-Tumorzellen und ihr Invasionspotential zu betsimmen, bspw. als Vitalitätstest für Stammzellen.

Die Offenbarung betrifft ferner folgende Gegenstände:
1. Verwendung einer Zusammensetzung zur Bestimmung des Invasions- und Metastasierungspotential von Tumorzellen, wobei als Zusammensetzung die Zusammensetzung der erfindungsgemäßen Verwendung verwendet wird,
2. Verfahren zur Kultivierung von biologischem Material, das folgende Schritte aufweist,
(1) Bereitstellung einer als Träger für biologisches Material geeigneten Zusammensetzung,
(2) Inkontaktbringen von biologischem Material mit der Zusammensetzung, und
(3) Inkubation der Zusammensetzung mit dem biologischen Material unter Kultivierungsbedingungen,
wobei als Zusammensetzung die Zusammensetzung der erfindungsgemäßen Verwendung verwendet wird.
3. Verfahren zur Implantation von biologischem Material in einen Organismus, das folgende Schritte aufweist,
(1) Bereitstellung einer als Träger für biologisches Material geeigneten Zusammensetzung,
(2) Inkontaktbringen von biologischem Material mit der Zusammensetzung, und
(3) Einbringen des biologischen Materials in Kontakt mit der Zusammensetzung in einen Organismus,
wobei als Zusammensetzung die Zusammensetzung der erfindungsgemäßen Verwendung verwendet wird.
4. Verfahren zur Verbesserung einer Zusammensetzung in ihrer Eignung zur Kultivierung von biologischen Zellen, die folgende Parameter ausweist:

| | |
|---|---|
| Kollagen [Gew.-%]: | ca. 30 bis 80, |
| Amidstickstoff [Gew.-%]: | ca. 0,06 bis 0,6, |
| Polyol [Gew.-%] | ca. 0 bis 50, |
| Fett [Gew.-%]: | ca. 0 bis 20, |
| Asche [Gew.-%]: | ca. 0 bis 10, |
| Wasser [Gew.-%]: | ca. 5 bis 40, |
| pH-Wert: | ca. 3 bis 10, |
| Flächengewicht [g/m²]: | ca. 10 bis 100, |
| Reißfestigkeit [N/mm²]: | ca. 0,5 bis 100, |

das folgende Schritte aufweist:
(1) Bereitstellung der Zusammensetzung, und
(2) Einstellung des pH-Wertes auf ca. 5,0 bis 8,0, vorzugsweise ca. 6,8 bis 8,0, weiter vorzugsweise auf ca. 7,0 bis 7,8, höchst bevorzugt auf ca. 7,2 bis 7,5.

5. Verfahren nach 4, wobei Schritt 2 folgenden Schritt umfasst:
(2.1) Inkubation der Zusammensetzung in Pufferlösung mit einem pH-Wert von ca. 7,2 bis 7,5.

6. Verfahren nach 4 oder 5, wobei es folgenden weiteren Schritt aufweist:
(3) Inkubation der Zusammensetzung mit stark fettlöslichen Substanzen.

7. Verfahren nach 6, wobei es folgenden weiteren Schritt aufweist:
(3.1) Waschen der Zusammensetzung in Pufferlösung.

8. Verfahren nach 4 bis 7, wobei es folgenden weiteren Schritt aufweist:
(4) Trocknung der Zusammensetzung.

9. Verfahren nach 4 bis 8, wobei es folgenden weiteren Schritt aufweist:
(5) Strahlungssterilisation der Zusammensetzung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, aus denen sich weitere Merkmale und Vorteile sowie Eigenschaften der Erfindung ergeben. Dabei wird Bezug auf die beigefügten Figuren genommen, in denen Folgendes zu sehen ist:
- Fig. 1: zeigt eine Kollageneinlage mit einer Stärke von 20 µm für eine Zellkulturplatte mit 6 Vertiefungen (A); eine erfindungsgemäße Schlauchhülle als Zellreservoir für Implantationsversuche (B, B'),
- Fig. 2: zeigt das Ergebnis eines BrdU-Proliferationsassays an humanen mesenchymalen Stammzellen (hMSC). Immuncytochemische Analyse der BrdU-positiven Zellen. Die Zellen wurden auf einer konventionellen Plastikkulturoberfläche (A, A') und auf der erfindungsgemäßen Kollagenmatrix (B, B') kultiviert und für eine Stunde mit BrdU inkubiert.
- Fig. 3: zeigt das Ergebnis des BrdU-Proliferationsassays (A) und des MTT-Tests (B) an humanen mesenchymalen Stammzellen (hMSC), die auf einer Kollagenmatrix und einer konventionellen Plastikkulturoberfläche kultiviert wurden. Dargestellt sind die Mittelwerte mit den jeweiligen Standardabweichungen.
- Fig. 4: zeigt eine Aufsichtsaufnahme auf eine mineralisierte Kollagenmatrix, die zusammen mit hMSCs unter osteogenen Differenzierungsbedingungen kultiviert wurde (A, A'); alkalische Phosphataseaktivität von embryonalen (E18) murinen Osteoblasten aus der Schädelkalotte nach einer zweiwöchigen Kultivierungsphase auf der Kollagenmatrix (B, B').
- Fig. 5: zeigt einen Paraffinquerschnitt durch die Kollagenmatrix, die mit embryonalen (E18) murinen osteogenen Progenitoren aus der Schädelkalotte kultiviert wurde, und zwar unter Proliferationsbedingungen (A, A') bzw. unter osteogenen Differenzierungsbedingungen (B, B'). Die Abbildung B bzw. B' verdeutlicht die durchgängige Mineralisierung der dreidimensionalen Matrix. Die Integrations- und Penetrationsleistung hängt vom Zelltyp ab.
- Fig. 6: zeigt die Implantation einer zellfreien erfindungsgemäßen Schlauchhülle in eine C57/BL6-Maus (A) und die implantierte Matrix nach 6 Wochen in der Nacktmaus (B).
- Fig. 7: zeigt die erfindungsgemäße Schlauchfolie direkt vor der Implantation, die für einen Tag mit hMSCs besiedelt wurde (A) und das explantierte, in das Bindegewebe eingewachsene Implantat nach 6 Wochen (B).
- Fig. 8: zeigt die erfindungsgemäße Schlauchfolie nach der Explantation nach 6 Wochen in einer C57/BL6-Maus in einer vergrößerten Darstellung. Deutlich sind die Blutgefäße erkennbar, die die Kollagenfolie durchziehen.
- Fig. 9: zeigt He-Färbungen an einem Paraffinschnitt, der vom explantierten Implantat angefertigt wurde.

### Ausführungsbeispiele

### 1. Kollagenhaltige Zusammensetzung

Die Erfinder haben sieben von der Firma Naturin GmbH & Co. KG kommerziell erhältliche Folien verwendet und festgestellt, dass diese für die erfindungsgemäße Verwendung geeignet sind. Die Parameter der getesteten, kommerziell erhältlichen Folien finden sich in nachstehender Tabelle 1:

**Tabelle 1: Parameter der von Naturin erhältlichen, auf Kollagen basierenden Folien**

| Zusammensetzung # | 1 | 2 | 3 | 4 | 5 | 6 | 7 |
|---|---|---|---|---|---|---|---|
| Referenznummer | 400011899 | 400023747 | 400024203 | 400026193 | 400019485 | 400000084 | 400000109 |
| Ausgestaltung der Zusammensetzung | Flachfolie | Schlauchhülle ∅ 110 mm | Schlauchhülle ∅ 140 mm | Schlauchhülle ∅ 60 mm | Schlauchhülle ∅ 40 mm | Schlauchhülle ∅ 65 mm | Schlauchhülle ∅ 115 mm |
| Kollagen [Gew.-%] | 65 | 76 | 79 | 76 | 79 | 77 | 77 |
| Wasser | 15 | 12 | 12 | 12 | 12 | 12 | 12 |
| Glycerin [Gew.-%] | 15 | 10 | 7 | 10 | 7 | 4 | 4 |
| Fett | | | | | | | |
| Acetoglycerid [Gew.-%] | 4 | - | - | - | - | - | - |
| vegetabiles Öl [Gew.-%] | - | 1 | - | 1 | 1 | 1 | 1 |
| Asche [Gew.-%] | 1 | 1 | - | 1 | 1 | 1 | 1 |
| pH-Wert | 5,1 | 3,4 | 3,4 | 3,4 | 3,4 | 4,8 | 4,8 |
| Dicke [µm] | 20 | 110 | 25 | 82 | 67 | 100 | 115 |
| Mikroskopierfähigkeit (Durchlicht/Fluoreszenz) | gut | möglich | nicht möglich | Fluoreszenz | Fluoreszenz | nicht möglich | Fluoreszenz |

### 1.1 Herstellung von erfindungsgemäßen Zusammensetzungen in Folienform

Als Ausgangsmaterial für die Herstellung erfindungsgemäßer Zusammensetzungen in Folienform dienen Rinderhautunterspalte, die hinsichtlich ihrer Rückverfolgbarkeit und den hygienischen Anforderungen den Bestimmungen der Verordnung (EG) Nr. 853/2004 genügen.

Diese Rinderhautunterspalte werden mechanisch grob vorzerkleinert und in mehreren Verfahrensschritten zunächst mit Wasser gewaschen und anschließend alkalisch aufgeschlossen. Der Aufschlussgrad kann variiert werden und hängt von Faktoren wie der Behandlungsdauer, der Konzentration des alkalischen Mediums (pH-Wert) und der Temperatur ab. In der Regel werden Kalkmilch oder Natronlauge oder Mischungen dieser beiden Komponente zur Einstellung des alkalischen Mediums verwendet, doch andere basische Verbindungen sind ebenfalls geeignet. Die Alkalibehandlung erfolgt bei einem pH-Wert von beispielsweise 12,5 und kann sich von beispielsweise 15 h bis über 150 h erstrecken, je nach beabsichtigter Intensität des Hautaufschlusses. Als ein möglicher Parameter zur analytischen Verfolgung des Aufschlussgrads des kollagenen Gewebes hat sich der Amidstickstoff bewährt: Je intensiver der Aufschluss, desto tiefer der Amidstickstoff.

Nach Erreichen des gewünschten Aufschlussgrads wird gesäuert und abschließend mehrmals mit Wasser gespült. Die Säuerung erfolgt in der Regel mit Salzsäure über einen Zeitraum von 6 - 10 h; dabei wird ein pH-Wert von < 2, vorzugsweise < 1 erreicht. Auch die Verwendung anderer Säuren ist möglich. Mittels mehrerer nachgeschalteter Waschvorgänge mit Wasser wird der pH-Wert abschließend auf 2,6 bis 3,3 angehoben.

Die resultierenden "Kollagenschwarten" werden daraufhin mechanisch durch Wolfen und Durchpressen des gewolften Materials durch Lochscheiben mit abgestuft kleineren Durchlassdimensionen zu einer gelartigen, viskoelastischen Masse verarbeitet.

### 1.1.1 Ausbildung als Flachfolie (Zusammensetzung Nr. 1)

Diese "konzentrierte" Kollagenmasse wird in einen Rührkessel überführt, in dem Glycerin, Wasser und Säure beigemischt werden. Gleichzeitig erfolgt hier die Einstellung des pH-Werts auf vorzugsweise 2,6 - 3,2 und der Gehalt an Trockenkollagen wird zwischen 1,6 Gew.-% und 2,5 Gew.-% eingestellt. Das Gemisch durchläuft anschließend einen Homogenisator, wird entlüftet und anschließend über eine Schlitzdüse auf ein Transportband gegossen, auf dem der resultierende Gel-Film einen Tunneltrockner durchläuft. Vor dem Einlaufen in den Trockner wird vorzugsweise mit Ammoniakgas begast und so der pH-Wert des Gels angehoben. Am Ende des Trockners durchläuft der trockene Film eine Wiederbefeuchtungszone, ehe er aufgewickelt wird.

### 1.1.2 Ausbildung als Schlauchhülle (Zusammensetzungen Nr. 2 bis 7)

Die viskoelastische Kollagenmasse von 1.1 wird in einen Knetmischer überführt, in dem rezepturabhängig Glycerin beigemischt wird. Gleichzeitig erfolgt durch Zugabe von Wasser und Säure die Einstellung von pH-Wert und Trockenstoffgehalt.

Anschließend wird die homogene Masse durch eine Ringschlitzdüse extrudiert. Dabei entsteht eine endlose Schlauchhülle. Durch gleichzeitiges Einblasen von Stützluft wird ein Kollabieren des Endlosschlauchs verhindert.

Der Transport der aufgeblasenen Schlauchhülle durch die Extrusionslinie verläuft je nach herzustellendem Darmtyp im Detail unterschiedlich. Prinzipiell besteht die Möglichkeit, chemikalienhaltige Duschbäder und Trocknersegmente in variabler Abfolge zu durchlaufen. Am Ende der Extrusionslinie wird die getrocknete Schlauchhülle zwischen Quetschwalzen flachgelegt und in diesem Zustand auf Spulen aufgewickelt.

Die so erhaltenen Schlauchfolien werden anschließend einer thermischen Behandlung unterzogen, wobei sie die für ihre spätere Verwendung benötigte mechanische Stabilität erhalten. Erfindungsgemäße Zusammensetzungen in Folien- oder Hüllenform können auch auf der Basis anderer Kollagenquellen gefertigt werden, wobei sich die Aufbereitung des Kollagengels in Details vom vorstehend Beschriebenen unterscheiden kann. Ausgehend von Schweinehautkollagen, beispielsweise, muss ein geeigneter Weg zur Reduzierung des Fettgehalts gefunden werden, der bspw. in der DE 100 60 643 und EP 1 423 016 beschrieben ist. Die Verwendung von Naturdärmen zur Herstellung einer Kollagenmasse ist beispielsweise in der ES 2 017 564 beschrieben. Diese Dokumente sind durch Inbezugnahme Teil der Offenbarung der vorliegenden Anmeldung.

### 1.2 Einstellung des pH-Wertes

Die Einstellung des pH-Wertes erfolgt unter Verwendung von calcium- und magnesiumhaltigem Phosphatpuffer [Phosphatgepufferte Saline (PBS) mit Ca⁺⁺ und Mg⁺⁺ (PAA H15-001)] der den pH-Wert der auf Kollagen basierenden Folie in den physiologischen Bereich von pH 7,2 bis pH 7,5 einstellt. Hierzu wird die Kollagenfolie unter Schütteln für 5 Tage mit dem Puffersystem gespült. Der Puffer wird zweimal täglich gewechselt.

Alternativ kann die Kollagenmembran auch für eine Stunde in einen glycerinhaltigen Phosphatpuffer mit einem pH-Wert von 7,3 eingetaucht werden (Phosphatpuffer: In 7722 g destilliertem Wasser werden 15,6 g KH₂PO₄, 71,3 g Na₂HPO₄ x 2 H₂O und 492,9 g Glycerin gelöst). Danach lässt man den so behandelten Film abtropfen und bringt ihn in einen Spannrahmen ein, in dem er über Nacht bei Raumtemperatur trocknet.

### 1.3 Weitere optionale Aufbereitung

Die Kollagenmembran wird nach einer kurzen Äquilibrierung in destilliertem Wasser mit 100 %igem Aceton zur Extraktion von fettlöslichen Substanzen und zum Ausfällen von wasserlöslichen Proteinen behandelt. Nach Entfernen des Acetons unter Unterdruck wird die trockene Membran dreimal für je eine Stunde mit calcium- und magnesiumhaltigem Phosphatpuffer (in g/l: KCl 0,2; KH₂PO4 0,2; NaCl 8,0; Na₂HPO₄wasserfrei 1,15; CaCl₂-2H₂O in H15-001 0,132; MgCl₂-2H₂O in H15-001 0,1) gewaschen. Zur Elimination der Puffersalze werden drei Waschvorgänge von je 1 Stunde in destilliertem Wasser vorgenommen.

### 1.4 Trocknung

Die erhaltene Membran bzw. Folie wird getrocknet. Dies kann in einem Trockenschrank bei 60°C erfolgen, wobei man Feuchtewerte von <5 %, bspw. 3 %, erzielen kann. Die Membran kann auch bei Raumtemperatur durch bloßes Liegenlassen an der Luft getrocknet werden, so dass sich eine letztlich von der relativen Luftfeuchtigkeit abhängige Ausgleichsfeuchte der Membran bzw. Folie einstellen wird, die in der Regel zwischen ca. 8 Gew.-% und ca. 13 Gew.-% liegt.

### 1.5 Formgebung und Strahlungssterilisation

Die erhaltenen getrockneten Kollagenmembranen bzw. Kollagenfolien können beliebig geschnitten oder gestanzt werden, z.B. in DINA5-Blätter. Diese lassen sich dann mittels Beta- oder Gammabestrahlung bei 25 kGy oder 50 kGy sterilisieren.

Die Kollagenfolie kann bspw. als Einlage für Plastikvertiefungsschalen jeglicher Bauart, bspw. Mikrotiterplatten, konfektioniert oder als vorzugsweise nahtlose Schlauchhülle mit Durchmessern von <2 mm, ca. 12 mm bis zu mehreren Zentimetern produziert werden. Ebenso ist ein thermisches Verschweißen oder Kleben der Folie möglich.

### 1.6 Parameter der hergestellten erfindungsgemäßen Zusammensetzungen in Folienform

In der nachstehenden Tabelle 2 sind die Parameter von verschiedenen Kollagenflachfolien dargestellt, die gemäß der vorstehend beschriebenen Vorgehensweise nach 1.1.1 erhalten wurden, wobei die Schritte gemäß Abschnitt 1.3 nicht durchgeführt wurden.

**Tabelle 2: Parameter der hergestellten auf Kollagen basierenden Folien;**

| Muster | A | B | C | D | E | F | G |
|---|---|---|---|---|---|---|---|
| Kollagen [Gew.-%] | 58 | 61 | 61 | 61 | 55 | 55 | 55 |
| Amidstickstoff [mMol/100g Trockenkollagen] | 28 | 37 | 37 | 37 | 31 | 31 | 31 |
| Glycerin [Gew.-%] | 16 | 25 | 25 | 25 | 30 | 30 | 30 |
| vegetabiles Öl [Gew.-%] | 5 | 0 | 0 | 0 | 0 | 0 | 0 |
| Sorbit [Gew.-%] | 3 | 0 | 0 | 0 | 0 | 0 | 0 |
| Asche (600°C); [Gew.-%] | 2 | 1 | 1 | 1 | 1 | 1 | 1 |
| Wassergehalt | 16 | 13 | 13 | 13 | 14 | 14 | 14 |
| pH-Wert | 5,2 | 7,0 | 7,0 | 7,0 | 7,1 | 7,1 | 7,1 |
| Flächengewicht [gm²] | 32 | 38 | 29 | 23 | 30 | 27,5 | 25 |
| Reißwert, längs [N/mm²] | 60 | 67 | 61 | 44 | 59 | 54 | 48 |
| Reißwert, quer [N/mm²] | 52 | 54 | 48 | 38 | 52 | 47 | 43 |
| Art der Sterilisation und Dosis | keine | (*) | (*) | (*) | (*) | (*) | (*) |

| | | | | | | | |
|---|---|---|---|---|---|---|---|
| (*) zu jedem der Muster 1 bis 6 gab es 5 Unterproben: Ohne Sterilisation (a), Beta-Strahlung 25 kGy (b), Beta-Strahlung 50 kGy (c), Gamma-Strahlung 25 kGy (d) und Gamma-Strahlung 50 kGy (e) | | | | | | | |

Folgende Analysemethoden wurden verwendet:
Kollagen über Hydroxiprolinbestimmung / Amidstickstoff analog EP1676595 (Geistlich Söhne AG) / Glycerin über HPLC / vegetabiles Öl durch Soxhlet-Extraktion / Sorbit über HPLC / Asche gravimetrisch nach Veraschen im Muffelofen, 5 h bei 600 °C) / Wassergehalt gravimetrisch nach Trocknen im Trockenschrank bei 105 °C / pH- Wert durch Kleinschneiden des Films, Einbringen der Schnipsel in eine 5 %-ige NaCl-Lösung und Messung mittels Glaselektrode nach 10 Minuten / Flächengewicht durch Wiegen eines 10 cm x 10 cm großen Filmstücks mit Ausgleichsfeuchte / Reißwerte längs und quer mittels einer UTS Universalprüfmaschine (Typ 3/205, UTS Testsysteme GmbH) nach Klimatisieren bei 21 °C / 60 % r.F. der ausgestanzten Prüfkörper und einer Traversengeschwindigkeit von 100 mm/min.

### 2. Kultivierung von biologischem Material

### 2.1 Ausgestaltung der Zusammensetzung

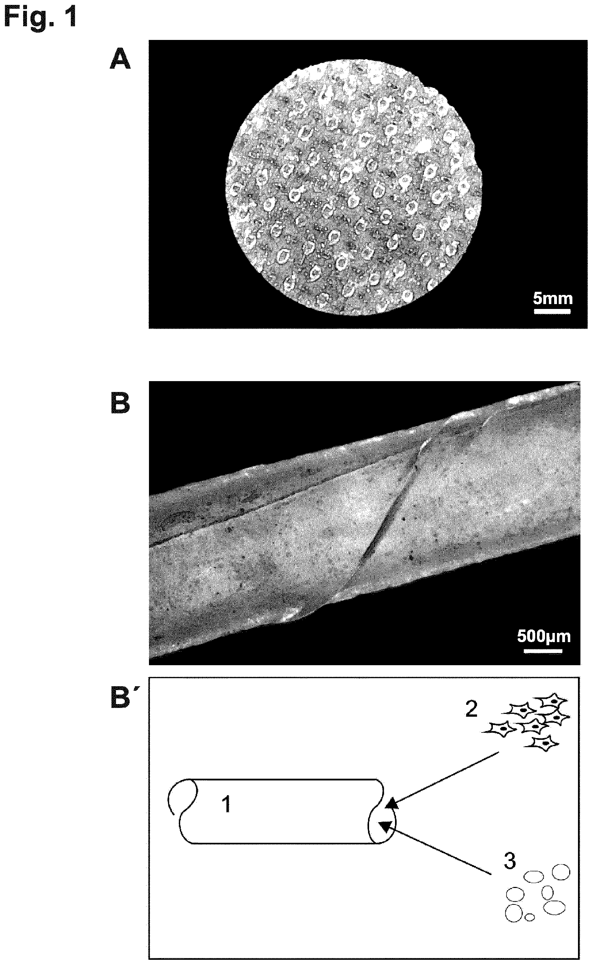

Fig. 1 zeigt eine erfindungsgemäße Kollagenfolie mit einer Dicke von 20 µm, ausgestaltet als Einlage für eine Vertiefung einer Zellkulturplatte (A). In Teilabbildung (B) ist eine Schlauchhülle abgebildet, die in Teilabbildung (B') schematisch dargestellt ist. Unter Bezugsziffer 1 ist die Schlauchhülle dargestellt. Unter Bezugsziffer 2 sind die Zellen dargestellt, die in das Innere der Hülle eingebracht werden können. Unter Bezugsziffer 3 sind ein Wirkstoff oder Wachstumsfaktoren dargestellt, die ebenfalls in die Hülle eingebracht werden können, um auf die biologischen Zellen einzuwirken.

### 2.2 Proliferationsverhalten von humanen Zellen

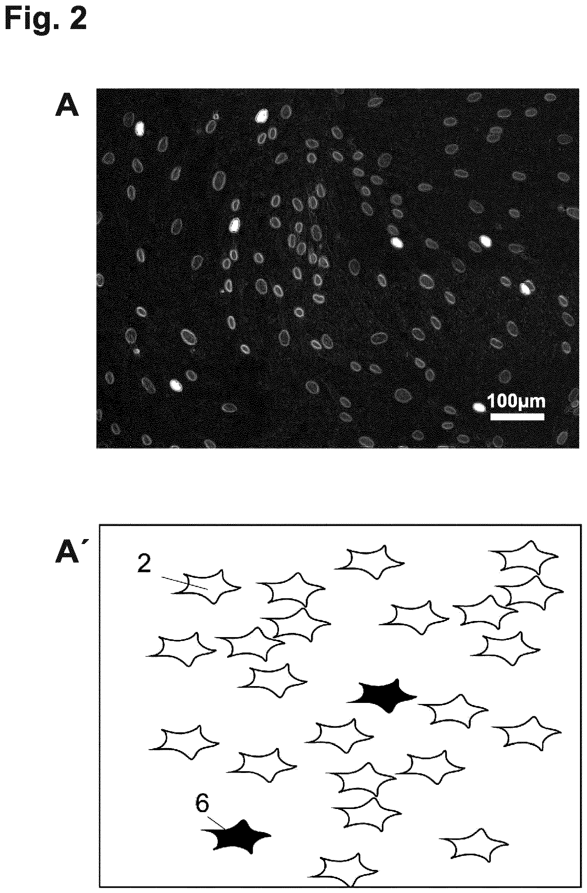

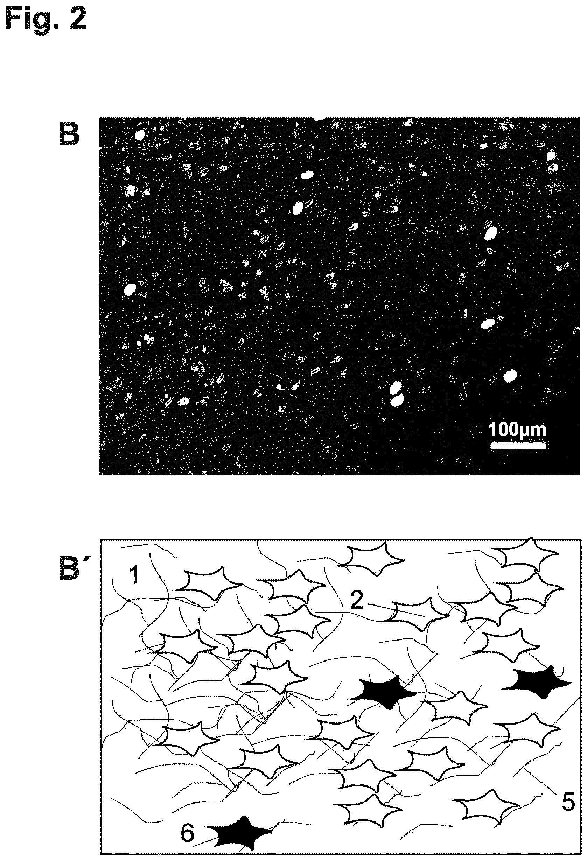

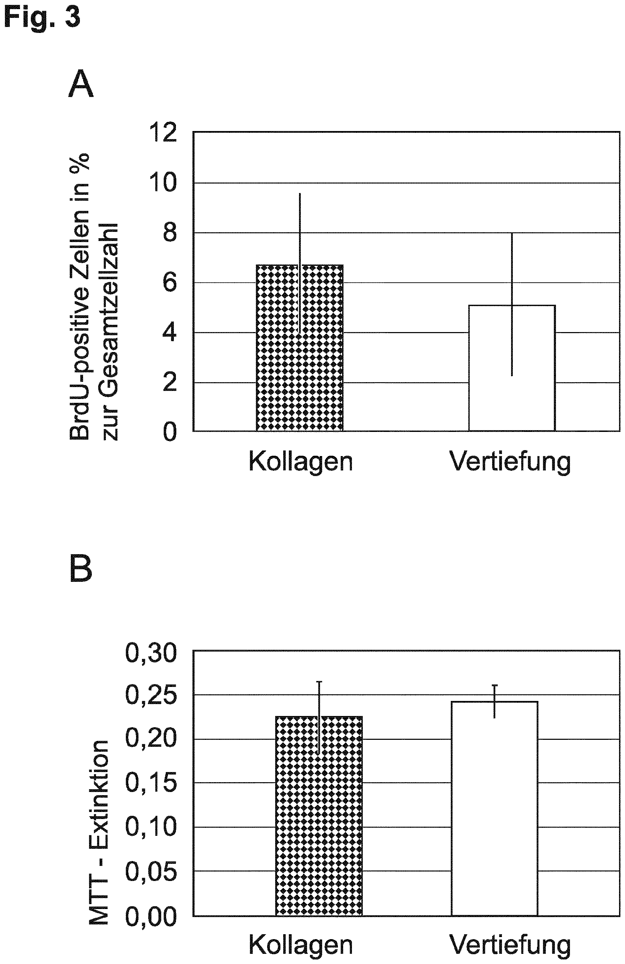

Das Proliferationsverhalten von humanen Zellen, mesenchymalen Stammzellen MSC und der humanen Zelllinie SaOS2, auf der erfindungsgemäßen Kollagenfolie zeigte keine Unterschiede gegenüber konventionellen Kultivierungsverfahren in der Plastikkulturschale; Fig. 2. Die Zellen wurden auf einer konventionellen Plastikkulturoberfläche (A) und auf der erfindungsgemäßen Kollagenfolie (B) kultiviert und für 1 Stunde mit BrdU inkubiert. In der schematischen Darstellung (B') ist mit 1 die Kollagenfolie, mit 2 die Zellen, mit 5 die Kollagenfasern und mit 6 die BrdU-positiven Zellen bezeichnet. Die statistische Auswertung des BrdU-Proliferationsassays (A) und MTT-Vitalitätstests (B) ist in Fig. 3 dargestellt.

Danach zeigen sich keine signifikanten Unterschiede zwischen der erfindungsgemäßen Kollagenfolie und den konventionellen Plastikschalen.

### 2.3 Biokompatibilität

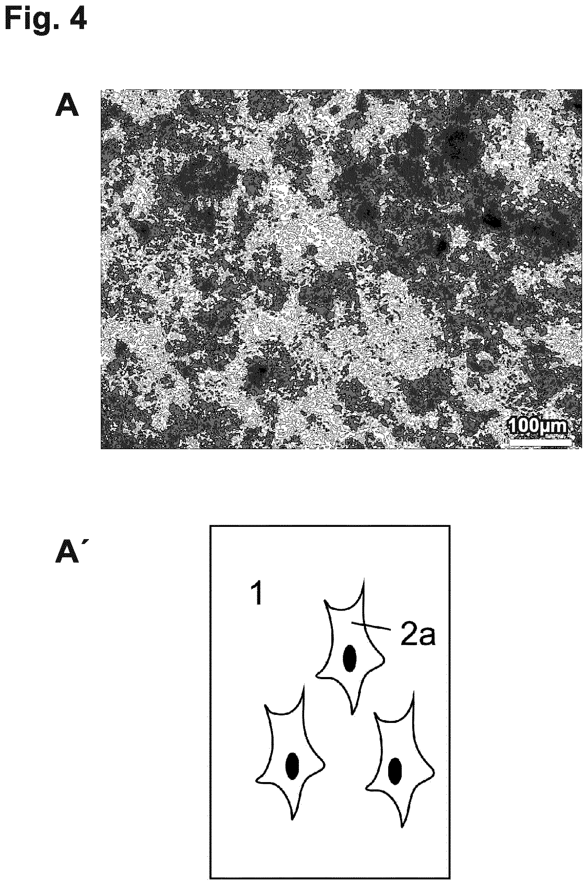

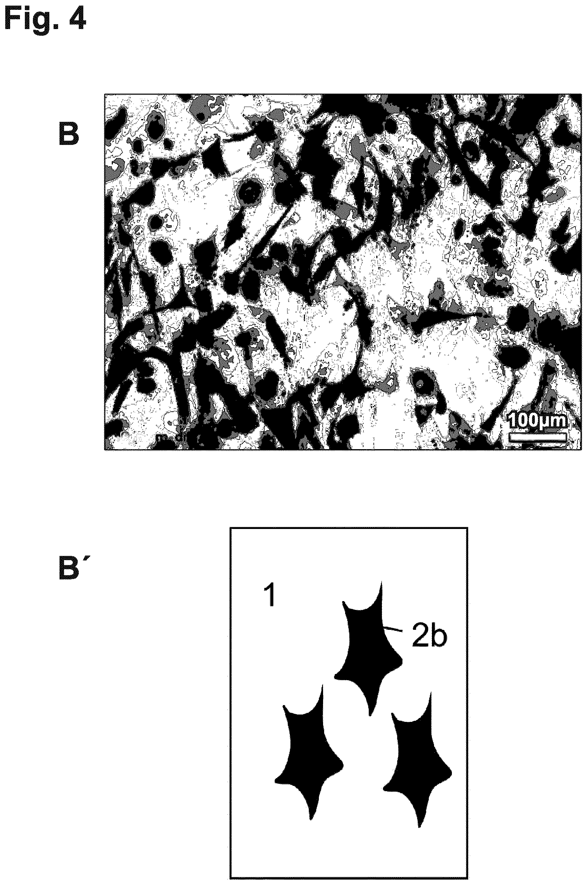

Zur Evaluierung der Biokompatibilität der erfindungsgemäßen Kollagenfolie wurden sowohl embryonale murine Progenitoren aus der Schädelkalotte als auch hMSCs auf dieser Matrix unter osteogenen Differenzierungsbedingungen kultiviert. Das Ergebnis ist in Fig. 4 dargestellt.

Teilabbildung (A) zeigt eine Aufsicht auf eine mineralisierte erfindungsgemäße Kollagenmatrix, die zusammen mit hMSCs unter osteogenen Differenzierungsbedingungen kultiviert wurde. Teilabbildung (B) zeigt die alkalische Phosphataseaktivität von embryonalen murinen Osteoblasten aus der Schädelkalotte nach einer 2-wöchigen Kultivierungsphase auf der erfindungsgemäßen Kollagenfolie. In den schematischen Teilabbildungen (A') bzw. (B') ist mit 1 die Kollagenmembran bezeichnet, mit 2a die Zellen und mit 2b die Zellen nach Detektion der zellulären alkalischen Phosphataseaktivität.

Der Nachweis der alkalischen Phosphataseaktivität und der zellinduzierten Mineralisierung verdeutlichen das Differenzierungspotenzial der kultivierten Zellen und somit die Biokompatibilität der erfindungsgemäßen Matrix.

### 2.4 Kultivierung von dreidimensionalen Gewebestrukturen

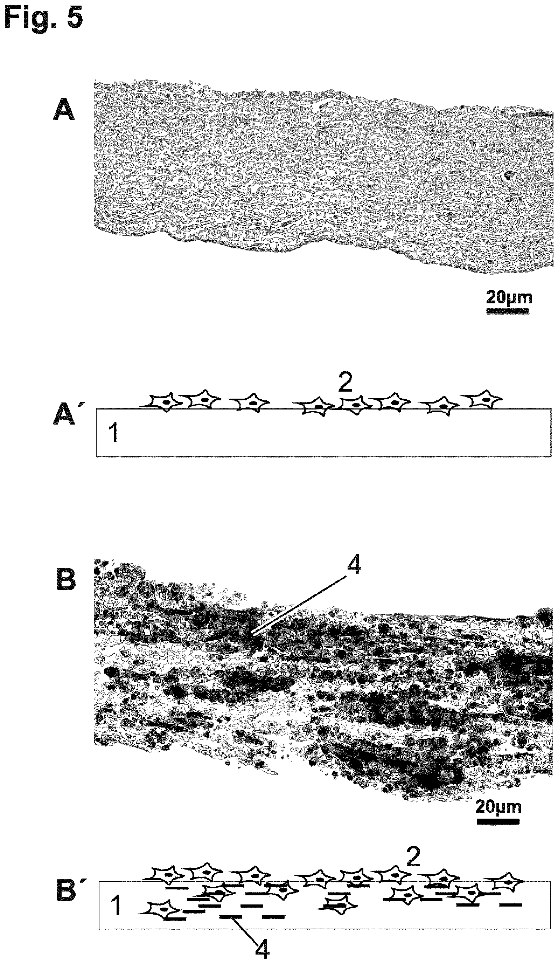

Von besiedelten Kollagenfolien wurden Paraffinquerschnitte angefertigt. Diese wurden histochemisch hinsichtlich der Mineralisierung analysiert. Das Ergebnis ist in Fig. 5 dargestellt. Teilabbildung (A, A') zeigt den Paraffinquerschnitt unter Proliferationsbedingungen, Teilabbildung (B, B') unter osteogenen Differenzierungsbedingungen. Mit 1 ist die Kollagenfolie bezeichnet, mit 2 die Zellen und mit 4 die Silbernitrateinlagerungen.

Dieses Experiment zeigt zum einen das hohe Mineralisierungspotenzial und zum anderen die Integrationsfähigkeit der Zellen innerhalb der dreidimensionalen Folie/Matrix. Mit Hilfe dieser erfindungsgemäßen Matrix ist die Züchtung von dreidimensionalen Gewebestrukturen denkbar.

### 2.5 Implantationen

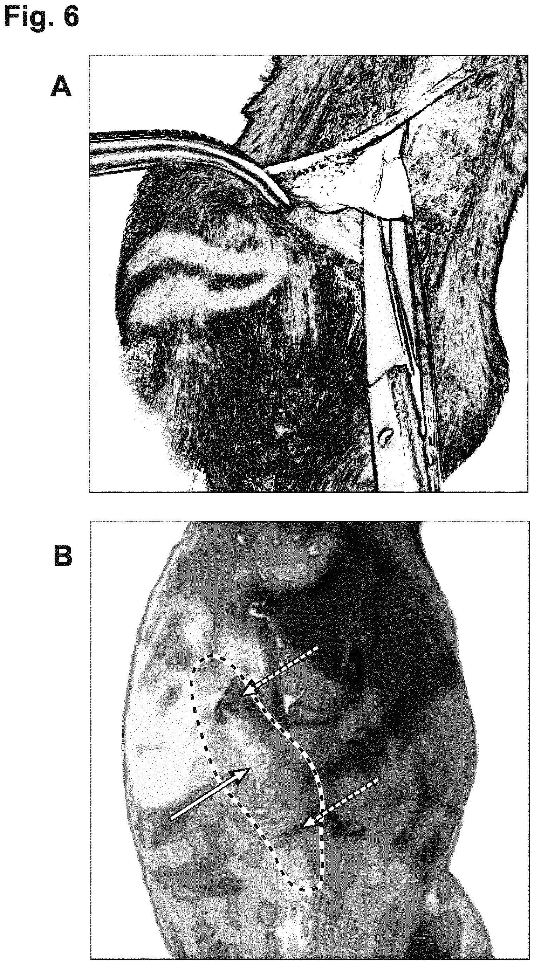

An Nackt- und C57/BL6-Mäusen wurden Implantationsexperimente durchgeführt. Dazu wurden zellbeladene erfindungsgemäße Schlauchhüllen im Bereich zwischen Subkutis und Peritoneum implantiert. Das Ergebnis dieses Experimentes ist in Fig. 6 gezeigt. Teilabbildung (A) zeigt die Implantation und Teilabbildung (B) die implantierte erfindungsgemäße Matrix nach 6 Wochen in der Nacktmaus. Der durchgezogene Pfeil deutet auf die zellbeladene erfindungsgemäße Schlauchhülle. In Teilabbildung (B) kann auch die mit nichtbiodegradierbaren Fäden eingebrachte Schlauchhülle gut anhand der Befestigungspunkte lokalisiert werden; gestrichelte Pfeile. Die Präparation zeigt in Teilabbildung (B) deutlich die noch vorhandene erfindungsgemäße Schlauchhülle.

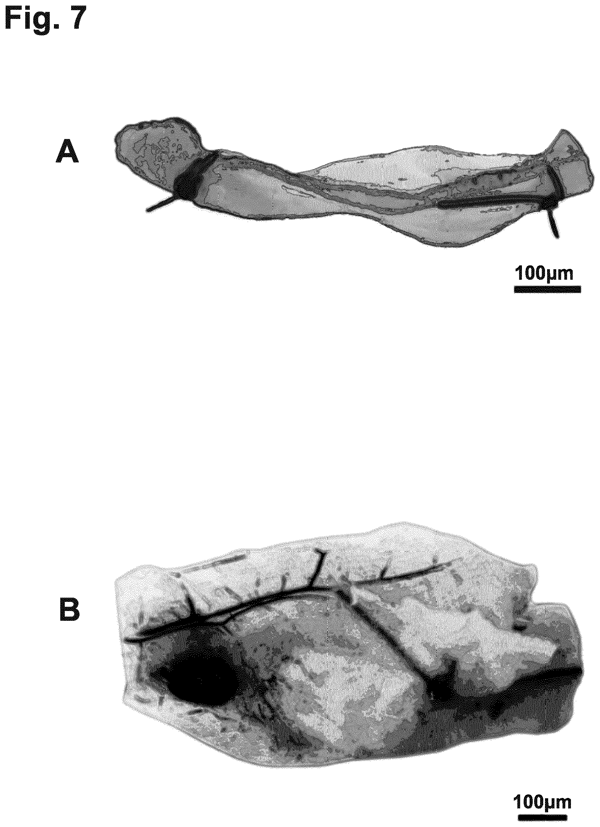

In Fig. 7 ist in Teilabbildung (A) die erfindungsgemäße Schlauchhülle direkt vor der Implantation dargestellt, die für einen Tag mit hMSCs besiedelt wurde. In Teilabbildung (B) ist das explantierte, in das Bindegewebe eingewachsene Implantat nach 6 Wochen dargestellt. Dabei zeigt sich, dass selbst 6 Wochen nach der Implantation die Integrität der Schlauchhülle trotz beginnender Resorptionsprozesse erhalten bleibt.

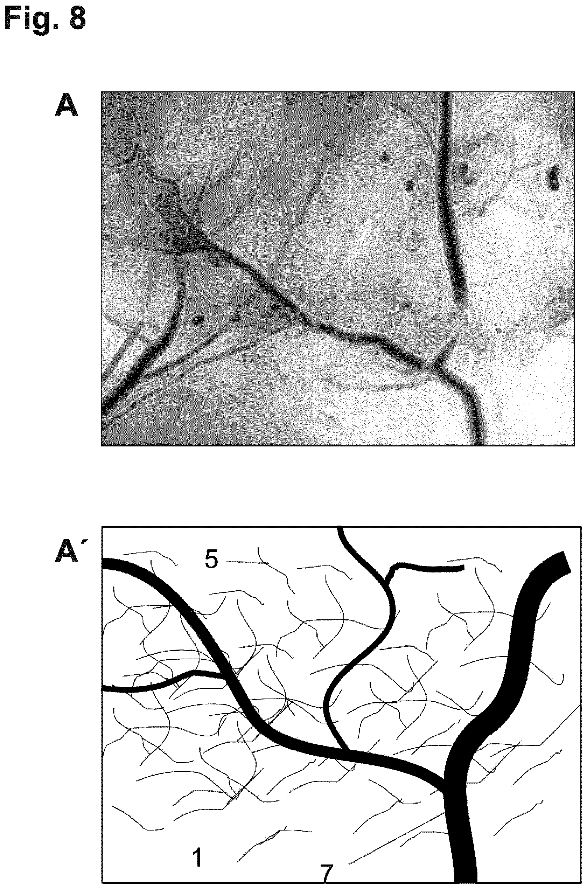

Das Implantat ist deutlich in das Bindegewebe des Tieres eingewachsen und wird von Blutgefäßen durchzogen; vgl. Fig. 8 (A, A'). In der schematischen Darstellung (A') sind mit 1 die Kollagenmembran, mit 2 die Zellen, mit 5 die Kollagenfasern und mit 7 die Blutgefäße bezeichnet.

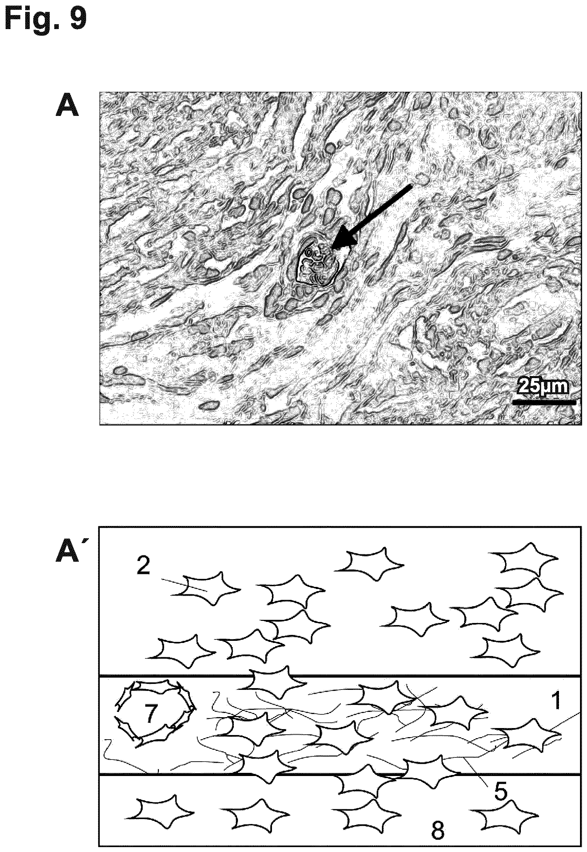

Immunhistologische Analysen an HE-gefärbten Paraffinschnitten zeigen Zellen, die in die erfindungsgemäße Folie eingewandert sind; vgl. Fig. 9. Deutlich ist ein Blutgefäß im Bereich des Implantates festzustellen (A, Pfeil). In der schematischen Darstellung (A') sind mit 1 die Schlauchhülle, mit 2 die Zellen, mit 5 die Kollagenfasern, mit 7 ein Blutgefäß und mit 8 Bindegewebe bezeichnet.

### 3. Fazit

Die Erfinder konnten eine kollagenhaltige Zusammensetzung, bspw. in Folien- oder Hüllenform bereitstellen, die reproduzierbar im Großmaßstab herstellbar ist und sich zur Kultivierung und Generierung von biologischem Material besonders gut eignet.

## Patentansprüche

1. Verwendung einer Zusammensetzung zur Kultivierung von biologischen Zellen, die folgende Parameter ausweist:
| | |
|---|---|
| Kollagen [Gew.-%]: | ca. 30 bis 80, |
| Amidstickstoff [Gew.-%]: | ca. 0,06 bis 0,6, |
| Polyol [Gew.-%] | ca. 0 bis 50, |
| Fett [Gew.-%]: | ca. 0 bis 20, |
| Asche [Gew.-%]: | ca. 0 bis 10, |
| Wasser [Gew.-%]: | ca. 5 bis 40, |
| pH-Wert: | ca. 3 bis 10, |
| Flächengewicht [g/m²]: | ca. 10 bis 100, |
| Reißfestigkeit [N/mm²]: | ca. 0.5 bis 100. |

2. Verwendung nach Anspruch 1, **dadurch gekennzeichnet, dass** das Polyol ausgewählt ist aus:
| | |
|---|---|
| Glycerin [Gew.-%]: | ca. 0 bis 50, und/oder |
| Sorbit [Gew.-%]: | ca. 0 bis 40. |

3. Verwendung nach einem der vorherigen Ansprüche, **dadurch gekennzeichnet, dass** das Fett im Wesentlichen pflanzliches Öl ist.

4. Verwendung nach einem der vorherigen Ansprüche, **dadurch gekennzeichnet, dass** der pH-Wert bei ca. 5,0 bis 8,0, vorzugsweise bei ca. 6,8 bis 8,0, weiter vorzugsweise bei ca. 7,0 bis 7,8, höchst bevorzugt bei ca. 7,2 bis 7,5 liegt.

5. Verwendung nach einem der vorherigen Ansprüche, **dadurch gekennzeichnet, dass** die Zusammensetzung als Flachfolie ausgebildet ist.

6. Verwendung nach einem der vorherigen Ansprüche, **dadurch gekennzeichnet, dass** die Zusammensetzung als Schlauchhülle ausgebildet ist.

7. Verwendung nach einem der vorherigen Ansprüche, **dadurch gekennzeichnet, dass** die Zusammensetzung in getrocknetem Zustand eine Dicke aufweist, die bei ca. 5 bis 200 µm liegt.

8. Verwendung nach Anspruch 7, **dadurch gekennzeichnet, dass** die Zusammensetzung in getrocknetem Zustand eine Dicke aufweist, die bei ca. 5 bis 100 µm, weiter bevorzugt bei ca. 10 bis 30 µm, höchst bevorzugt bei ca. 15 µm liegt.

9. Verwendung nach einem der vorherigen Ansprüche, **dadurch gekennzeichnet, dass** die Zusammensetzung als Träger für Zelfkultivierungsanwendungen ausgebildet ist.

10. Verwendung nach einem der vorherigen Ansprüche, **dadurch gekennzeichnet, dass** die Zusammensetzung als Träger für die Implantation von biologischem Material, vorzugsweise von Stamm- Vorläuferzellen, in einen Organismus ausgebildet ist.

11. Verwendung nach einem der vorherigen Ansprüche, **dadurch gekennzeichnet, dass** die Zusammensetzung strahlungssterilisiert ist.

12. Verwendung nach Anspruch 11, **dadurch gekennzeichnet, dass** die Strahlungssterilisation durch ionisierende Strahlung erfolgt.

13. Verwendung nach Anspruch 142, **dadurch gekennzeichnet, dass** die ionisierende Strahlung Beta-Strahlung und/oder Gamma-Strahlung ist.

14. Verwendung nach einem der vorherigen Ansprüche, **dadurch gekennzeichnet, dass** die erfindungsgemäße Zusammensetzung einen Farbstoff, vorzugsweise einen fluoreszenzabsorbierenden Farbstoff, aufweist.

## Claims

1. Use of a composition for the cultivation of biological cells, comprising the following parameters:
| | |
|---|---|
| Collagen [wt. -%]: | approx. 30 to 80, |
| Amide nitrogen [wt. -%]: | approx. 0 to 0.6, |
| Polyol [wt. -%]: | approx. 0 to 50, |
| Fat [wt. -%]: | approx. 0 to 20, |
| Ash [wt. -%]: | approx. 0 to 10, |
| Water [wt. -%]: | approx. 5 to 40, |
| pH Value: | approx. 3 to 10, |
| Weight per unit area [g/m²]: | approx. 10 to 100, |
| Tensile strength [N/mm²]: | approx. 0.5 to 100 |

2. Use of claim 1, **characterized in that** the polyol is selected from:
| | |
|---|---|
| Glycerin [wt. -%]: | approx. 0 to 50, and/or |
| Sorbite [wt. -%]: | approx. 5 to 40. |

3. Use of any of the preceding claims, **characterized in that** the fat is essentially vegetable oil.

4. Use of any of the preceding claims, **characterized in that** the pH value is at approx. 5.0 to 8.0, preferably at approx. 6.8 to 8.0, more preferably at approx. 7.0 to 7.8, most preferably at approx. 7.2 to 7.5.

5. Use of any of the preceding claims, **characterized in that** the composition is configured as a flat film.

6. Use of any of the preceding claims, **characterized in that** the composition is configured as a tubular casing.

7. Use of any of the preceding claims, **characterized in that** the composition in a dry condition comprises a thickness of approx. 5 to 200 µm.

8. Use of claim 7, **characterized in that** the composition in a dry condition state comprises a thickness of approx. 5 to 100 µm, more preferably of approx. 10 to 30 µm, most preferably of approx. 15 µm.

9. Use of any of the preceding claims, **characterized in that** the composition is configured as a carrier for applications of cell cultivation.

10. Use of any of the preceding claims, **characterized in that** the composition is configured as a carrier for the implantation of biological material, preferably of stem precursor cells, into an organism.

11. Use of any of the preceding claims, **characterized in that** the composition is sterilised through radiation.

12. Use of claim 11, **characterized in that** said radiation sterilisation is carried out using ionising radiation.

13. Use of claim 12, **characterized in that** the ionising radiation is beta-radiation and/or gamma-radiation.

14. Use of any of the preceding claims, **characterized in that** the composition comprises a dye, preferably a fluorescence-absorbing dye.

## Revendications

1. Utilisation d'une composition servant à la culture de cellules biologiques, qui présente des paramètres suivants :
du collagène [% en poids] : environ 30 à 80,
de l'azote d'amide [% en poids] : environ 0,06 à 0,6,
du polyol [% en poids] : environ 0 à 50,
de la matière grasse [% en poids] : environ 0 à 20,
des cendres [% en poids] : environ 0 à 10,
de l'eau [% en poids] : environ 5 à 40,
une valeur pH : environ 3 à 10,
un poids surfacique [g/m²] : environ 10 à 100,
une résistance au déchirement [N/mm²] : environ 0,5 à 100.

2. Utilisation selon la revendication 1, **caractérisée en ce que** le polyol est choisi parmi :
de la glycérine [% en poids] : environ 0 à 50, et/ou
du sorbitol [% en poids] : environ 0 à 40.

3. Utilisation selon l'une quelconque des revendications précédentes, **caractérisée en ce que** la matière grasse est sensiblement une huile végétale.

4. Utilisation selon l'une quelconque des revendications précédentes, **caractérisée en ce que** la valeur pH est de l'ordre d'environ 5,0 à 8,0, de préférence de l'ordre d'environ 6,8 à 8,0, encore plus préférentiellement de l'ordre d'environ 7,0 à 7,8, de manière plus préférée de l'ordre d'environ 7,2 à 7,5.

5. Utilisation selon l'une quelconque des revendications précédentes, **caractérisée en ce que** la composition est réalisée sous la forme d'un film plat.

6. Utilisation selon l'une quelconque des revendications précédentes, **caractérisée en ce que** la composition est réalisée sous la forme d'une enveloppe tubulaire.

7. Utilisation selon l'une quelconque des revendications précédentes, **caractérisée en ce que** la composition présente dans un état sec une épaisseur qui est de l'ordre d'environ 5 à 200 µm.

8. Utilisation selon la revendication 7, **caractérisée en ce que** la composition présente dans un état sec une épaisseur qui est de l'ordre d'environ 5 à 100 µm, de manière davantage préférée de l'ordre d'environ 10 à 30 µm, de manière préférée entre toutes de l'ordre de 15 µm.

9. Utilisation selon l'une quelconque des revendications précédentes, **caractérisée en ce que** la composition est réalisée sous la forme d'un support pour des applications de culture de cellules.

10. Utilisation selon l'une quelconque des revendications précédentes, **caractérisée en ce que** la composition est réalisée sous la forme d'un support pour l'implantation d'un matériau biologique, de préférence de cellules souches précurseurs, dans un organisme.

11. Utilisation selon l'une quelconque des revendications précédentes, **caractérisée en ce que** la composition est stérilisée par irradiation.

12. Utilisation selon la revendication 11, **caractérisée en ce que** la stérilisation par irradiation est effectuée par un rayonnement ionisant.

13. Utilisation selon la revendication 12, **caractérisée en ce que** le rayonnement ionisant est un rayonnement bêta et/ou un rayonnement gamma.

14. Utilisation selon l'une quelconque des revendications précédentes, **caractérisée en ce que** la composition selon l'invention présente un colorant, de préférence un colorant absorbant la fluorescence.
